# EUROPEAN PATENT SPECIFICATION

(11) **EP 2 310 422 B1**
(45) Date of publication and mention of the grant of the patent: **13.12.2017**
(21) Application number: 09800059.9
(22) Date of filing: 22.07.2009
(51) Int. Cl.: C08B 37/08, C08L 5/08

(54) **NANOCOMPOSITE MATERIALS BASED ON METALLIC NANOPARTICLES STABILIZED WITH BRANCHED POLYSACCHARIDES**
NANOKOMPOSITE AUF DER BASIS VON MIT VERZWEIGTEN POLYSACCHARIDEN STABILISIERTEN METALLISCHEN NANOPARTIKELN
MATÉRIAUX NANOCOMPOSITES À BASE DE NANOPARTICULES MÉTALLIQUES STABILISÉES AVEC DES POLYSACCHARIDES RAMIFIÉS

(30) Priority: 23.07.2008 IT PD20080219
(43) Date of publication of application: 20.04.2011
(73) Proprietor: Universita'Degli Studi di Trieste, I-34127 Trieste (IT)
(72) Inventor: DONATI, Ivan, I-33039 Sedegliano (IT); MARSICH, Eleonora, I-34148 Trieste (IT); TRAVAN, Andrea, I-34100 Trieste (IT); PAOLETTI, Sergio, I-34136 Trieste (IT)
(74) Representative: Gervasi, Gemma
(86) International application number: PCT/EP2009/059430
(87) International publication number: WO 2010/010122

(56) References cited:
- WO-A-2007/135114
- US-A1- 2008 147 019
- HUANG ET AL: "Preparation and characterization of metal-chitosan nanocomposites" COLLOIDS AND SURFACES. B, BIOINTERFACES, ELSEVIER, AMSTERDAM, NL, vol. 39, no. 1-2, 25 November 2004 (2004-11-25), pages 31-37, XP005003635 ISSN: 0927-7765
- YANG T C ET AL: "Antibacterial activity of N-alkylated disaccharide chitosan derivatives", INTERNATIONAL JOURNAL OF FOOD MICROBIOLOGY, ELSEVIER BV, NL, vol. 97, no. 3, 1 January 2005 (2005-01-01), pages 237-245, XP027663009, ISSN: 0168-1605 [retrieved on 2005-01-01]

## Description

### Field of the invention

The present invention relates to nanocomposite materials formed by metallic nanoparticles stabilized in a matrix of branched cationic polysaccharides, preparation and use thereof for applications in biomedical, pharmaceutical and food field.

### State of the art

The preparation of metallic nanoparticles is a research area of high nano-technological interest; indeed, many metals have particular optical, antimicrobial and catalytic properties typically associated with the nano-scale. In particular, the broad spectrum antimicrobial activity of metals, such as for example silver, has a remarkable application interest. On the other hand, bacterial, fungal and viral infections represent a serious problem in many situations and thus the implementation of products with a broad antimicrobial spectrum is a particularly felt need. In fact, the use of metals such as silver, gold, copper, zinc and nickel in antimicrobial material field is having a great influence on the market, especially for treating topic tissue lesions, so that, for example, companies such as Johnson&Johnson® and Convatec® have recently marketed medications which are based on antibacterial properties of silver nanoparticles.

Nanoparticles are generally obtained from metal salt solutions in the presence of a reducing agent and a stabilizing agent, which serves to prevent nanoparticles from aggregating. In order to stabilize the nanoparticles, appropriate diluted polymeric solutions are widely used, which allow to obtain nanocomposite systems in which the metallic particles result to be homogeneously dispersed. For the above-mentioned applications, solutions of polyelectrolytes, such as polyphosphate, polyacrylate, poly(vinyl sulphate), poly(allylamine) (Henglein, A., J. Phys. Chem. 1993, 97 (21), 5457-5471), poly(ethylenimine) (Kuo, P. L.; Chen, W. F. J. Phys. Chem. B 2003, 107 (41), 11267-11272; Dai et al. (Nano Lett. 2002, 2 (5), 497-501) were particularly effective.

In general, the components required to obtain the formation of metallic nanoparticles are: a suitable metal salt, a reducing agent and a polymer acting as a stabilizer for the colloidal suspension.

US 2007/00036031 (Karandikar et al.) discloses methods for the formation of silver nanoparticles in the presence of a solvent, a stabilizing agent, a surfactant, a reducing agent and heating. Among the stabilizing agents, polymers such as polyacrylamide and polysorbate 20 are mentioned.

In patent WO2007/147094A2 (Sambhy V. et al.) nanocomposite systems in synthetic cationic polymeric matrices, characterized by a ternary or quaternary nitrogen atom, in which the nanoparticles-stabilizing polymer is a poly(4-vinyl pyridine) derivative and silver bromide is employed to form these silver nanoparticles, are described.

Patent WO2007/017901A2 (Omray P. et al.) reports formulations of silver nanoparticles dispersed, for example, by using polysorbate, propylene glycol, and propyl gallate.

In patent WO2007/096606A1 (Crowther N. et al.) methods for releasing metallic nanoparticles, prepared in the presence of stabilizing polymers for antimicrobial applications, are described. Among the mentioned polymers there are: poly(methacrylic acid), polyimides, poly(vinylalcohol) and copolymers thereof. Patent WO2007/001453 (Yacaman M. et al.) reports the silver nanoparticle synthesis in the presence of polyols and polymers by using glycerin or ethylene glycol both as reducing agent and as nanostructure solvent and by using poly(vinylpyrrolidone) as coating agent for nanoparticles. It is noted that the chemical reduction mechanism of silver ions is based on the oxidation of hydroxyl groups present in polyols and breakdown of C-C bonds.

For applications in the biomedical field, systems based on polysaccharides are particularly interesting because these polymers are generally biocompatible and therefore suitable for applications that involve a direct contact with biological tissues. Among the natural polysaccharides, one of the more studied and commercially employed is chitosan. This is a basic polysaccharide, with a molecular weight from 50 to 1'500 kDa, consisting of a straight chain of D-glucosamine (GlcNH2) residues bonded by β1→4 bonds with interspersed residual N-acetyl-glucosamine units from the incomplete chitin de-acetylation. This polysaccharide is usually insoluble in neutral or basic aqueous solutions; in acidic solutions with pH equal or less than 5, the free amino group is protonated, thus making the polymer soluble. This polymer is already widely employed in medical field as it exhibits a low immunogenic, pathologic or infective response (Suh Francis J.K., Matthew H.W.T. Biomaterials, 2000, 21, 2589-2598; Miyazaki S. et al. Chem. Pharm. Bull., 1981, 29, 3067-3069). Chitosan has all ideal features to be employed as a biomaterial due to its physical-chemical properties, such as high cationic charge density in acidic solution, its high processability and the ability to give rise to porous structures where, for example, cells may be implanted. Indeed, many uses of chitosan, both in biomedical and alimentary field are known. Due to these properties, both chitosan and its derivatives were also used in order to stabilize nanoparticles.

In patent WO2007/025917A1 (Schmid H. et al.) silver nanoparticle preparations in the presence of chitosan as well as chitosan derivatives and salts such as carboxymethylchitosan, chitosan acetate and chitosan lactate, are mentioned.

In the patent US 2008/0147019 (Song Xuedong), commercially available preformed metal nanoparticles are added to chitosan salts and chitosan derivatives, but not branched derivatives of saccharidic nature; these nanoparticles require further oxidation to provide for an antibacterial activity since the silver ions (Ag⁺) are pointed as the only antibacterial agent.

In the patent WO2008/076339 (Schauer C et al), commercially available metal nanoparticles are included in water-insoluble cross-linked chitosan based materials. The reticulated chitosans are used to prepare multilayer films to support nanoparticles which are used as coloring agents and as sensors for detecting contaminants in water.

Several recent researches have focused on improving methods for enhancing the biological effects of chitosan. In particular, most efforts were directed to increase the cationic feature of the polymer or modify the chemical features and bioavailability thereof by means of (bio)chemical modifications.

In particular, chitosan modification with saccharidic groups, for example by inserting lactose units by means of a reductive amination reaction resulted, as mentioned in US 4,424,346 (Hall, L.D. and Yalpani, M.), in higher water solubility of chitosan derivatives and it is indeed in these derivatized forms that chitosan acquires the most useful properties for use as a highly biocompatible biomaterial.

### Summary

It is a first object of the present invention to obtain a nanocomposite material, where metallic nanoparticles are stabilized and size-controlled and the properties of which are particularly suitable for applications in biomedical and optical (biosensors) field.

It is a further object that such a nanocomposite is obtainable by means of a non-complex and economically advantageous chemical approach, and in particular by colloidal solutions in which metallic nanoparticles are stabilized by appropriate polysaccharidic solutions.

It is a further object to improve these systems by employing readily commercially available polysaccharides and avoiding these polysaccharides from being subjected to chemical manipulations, as well as avoiding the need for complex preparation manipulations of these systems.

It is a further object to obtain systems that are totally soluble in aqueous systems in the conditions of neutral pH and substantial ionic strength that are required in biomedical applications.

In order to achieve the above-mentioned objects, the inventors developed suitable soluble polysaccharidic systems based on branched cationic polysaccharides which allow to obtain stable, size-controlled metallic nanoparticles. The procedures are based on mixing aqueous polymeric solutions and aqueous solutions of metal salts in the presence or absence of exogenous reducing agents. The mixing procedures can be conveniently carried out in aqueous systems at neutral pH and does not require heating, since it can be easily carried out at room temperature.

Therefore, in a first aspect, an object of the present invention is given by nanocomposite materials according to claim 1, characterized in that they comprise a polymeric matrix consisting of branched cationic polysaccharides and metallic nanoparticles dispersed in such a polymeric matrix.

Preferred cationic polysaccharides are branched derivatives of chitosan, wherein the D-glucosamine units forming the linear chitosan chain bind, by means of the functional group -NH- on carbon atom C2, the alditolic or aldonic polyols residues equal to or different from one another represented by the general formula (**I**): 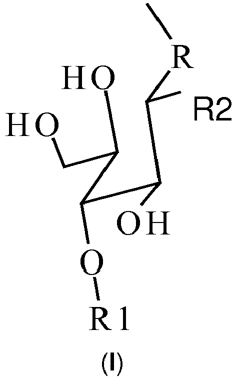 where:
- R is -CH₂- or -CO-;
- R₁ is hydrogen, a monosaccharide, an oligosaccharide;
- R₂ is -OH or -NHCOCH₃.

On the other hand, the metals of nanoparticles are preferentially silver, gold, platinum, palladium, copper, zinc or nickel and mixtures thereof.

Nanocomposite materials according to the invention can be prepared under appropriate conditions with aqueous solutions of these polysaccharides and metal salts in the presence or absence of exogenous reducing agents.

Therefore, according to another aspect, it is an object of the invention a method of preparing these nanocomposite materials comprising the steps of:
a) preparing aqueous solutions of branched cationic polysaccharides at a concentration up to 2% w/v;
b) preparing aqueous solutions of metal salts at a concentration between 0.1 mM and 14 mM;
c) adding these salt solutions to solutions of polysaccharides and mixing up to reduction of the metal ions and formation of colloidal solutions of metal nanoparticles wherein said polysaccharide -reduced metallic nanoparticles are homogeneously dispersed.

A reducing agent is optionally added to the obtained colloidal solutions.

According to a further aspect, the use of these nanocomposite materials in biomedical, pharmaceutical and alimentary field, for example as antimicrobial agents, or in molecular biosensors, is another object of the invention.

### Brief description of the figures

Figure 1. The figure shows the stabilization of silver nanoparticles in chitosan derivative with lactose (hereinafter also referred to as Chitlac; CAS registry number 85941-43-1): the polymeric chains of Chitlac allows the coordination and stabilization of metallic nanoparticles. TEM images, obtained by means of a Philips EM 208 transmission electronic microscope, demonstrate that silver nanoparticles (black particles) are exactly located on polymeric chains (gray branched structures). Sample prepared by using chitlac (0.2% w/v) and 1 mM AgNO3 (Example 7).

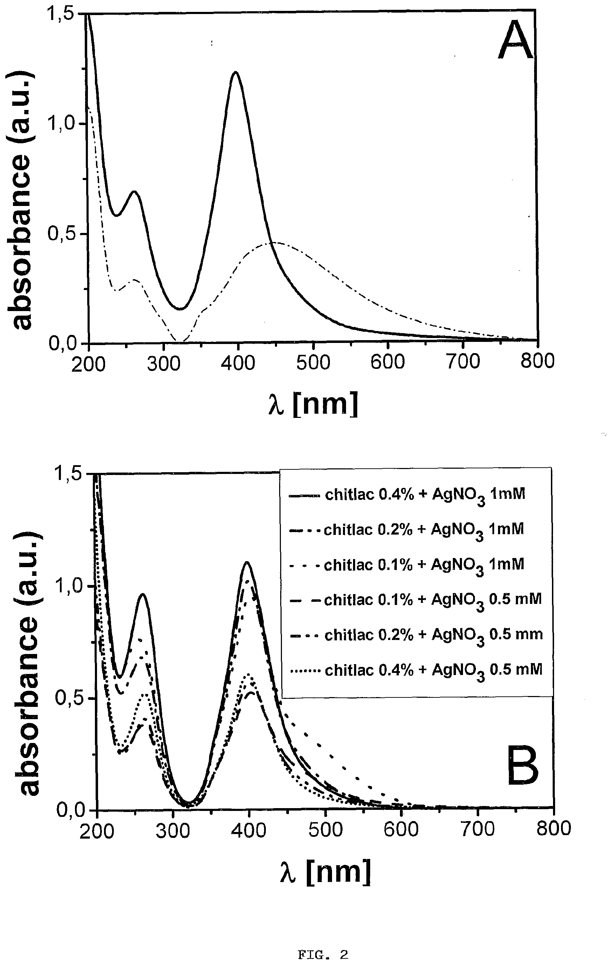

Figure 2. The figure shows: **(A)** UV-Vis spectra of silver nanoparticles formed in the presence of chitosan (broken line, Example 22) and Chitlac (continuous line, Example 7) under the same conditions (0.2 % w/v polymer; 1 mM AgNO₃; 0.5 mM ascorbic acid (C₆H₈O₆)); **(B)** Effect of the concentrations of Chitlac and AgNO₃ on UV-Vis spectra: 0.4% w/v Chitlac/ 1 mM AgNO₃ (continuous line Example 9); 0.2% w/v Chitlac/ 1 mM AgNO₃ (broken line Example 7); 0.1 % w/v Chitlac/1 mM AgNO₃ (spaced-dot line ^{...} Example 5); 0.1% w/v Chitlac/0.5 mM AgNO₃ (close-dot line ^{...} Example 4); 0.2% w/v Chitlac/0.5 mM AgNO₃ (stroke-dot line ^{-.-.-} Example 6); 0.4% w/v Chitlac/0,5 mM AgNO₃ (stroke-dot-dot line ^{-..-..} Example 8). According to the reaction stoichiometry, in each sample the AgNO₃/C₆H₈O₆ ratio is 2. The measurement was carried out by means of a CaryE4 UV-visible spectrophotometer at a temperature of 25 °C.
Figure 3. The figure shows the UV-Vis spectrum of gold nanoparticles formed in Chitlac in the absence of exogenous reducing agents (0.2 % w/v polymer; 1 mM HAuCl₄, Example 18). The measurement was carried out as previously described in Figure 2.
Figure 4. The figure shows UV-Vis spectra of samples of 0.2% w/v Chitlac/Agₙ (with silver nanoparticles) at different silver concentrations in the presence and absence of ascorbic acid. The samples were prepared according to the procedures described in examples: 7 (1 mM AgNO₃/0.5 mM C₆H₈O₆, continuous line), 10 (5 mM AgNO₃, line ^{-·-·-}), 11 (10 mM AgNO₃, line ^{-··-··-}), 25 (0.2% w/v Chitlac, 1 mM AgNO₃, line ---). The measurements were carried out as previously described in Figure 2.
Figure 5. TEM image of silver nanoparticles formed in Chitlac in the absence of exogenous reducing agents (0.4% w/v Chitlac, 14 mM AgNO₃, Example 15).
Figure 6. The figure shows the TEM image of gold nanoparticles dispersed in Chitlac (0.2% w/v Chitlac, 1 mM HAuCl₄, Example 18) in the absence of exogenous reducing agents. The measurement was carried out as previously described in Figure 1.
Figure 7. The figure shows the size distribution of gold nanoparticles dispersed in Chitlac (0.2% w/v Chitlac, 1 mM HAuCl₄, Example 18). The measurement was carried on by using a Nanosight LM20 system. The particle size is in the range from 10 to 150 nm and the most particles have dimensions around 50 nm.
Figure 8. The figure shows the TEM image of copper nanoparticles dispersed in Chitlac (0.2% w/v Chitlac, 1 mM CuSO₄, 0.2 mM NaBH₄, Example 19). The measurement was carried out as previously described in Figure 1.
Figure 9. The figure shows TEM images **(A, B)** and size distribution **(C)** of silver nanoparticles dispersed in Chitlac (0.2% w/v Chitlac, 1 mM AgNO₃, 0.5 mM C₆H₈O₆, Example 7). The measurement was carried out as previously described in Figure 1.

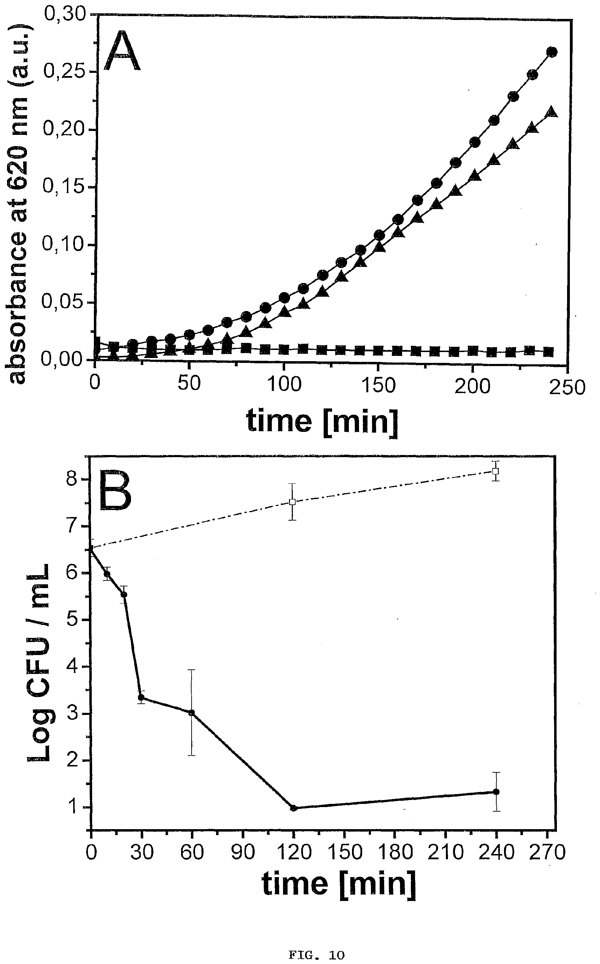

Figure 10. The figure shows: **(A)** bacterial growth kinetic of *E. coli ATCC* 25922 in 20% Mueller-Hinton culture medium (20% Mueller-Hinton; control, triangles) or in the presence of 0.2% w/v Chitlac (circles, Example 1) or in 0.2% w/v Chitlac + 1 mM AgNO₃; + 0.5 mM C₆H₈O₆ (squares, Example 7), respectively. The reported data refer to three independent experiments with comparable result; **(B)** Count of *E. coli* ATCC 25922 colonies in 0.2% w/v Chitlac + 1 mM AgNO₃; + 0.5 mM C₆H₈O₆ (continuous line, Example 7). The broken line indicates controls in the absence of Chitlac-nAg. The results are average (±SD) of at least 4 independent experiments.
Figure 11. The figure shows the bacterial growth kinetic of *E. coli* ATCC 25922 in Mueller-Hinton culture medium (20% Mueller-Hinton; control, circles) or in 0.2% w/v Chitlac + 1 mM HAuCl₄ (Chitlac-nAu, squares, Example 18).
Figure 12. The figure shows the count of *E. coli* ATCC 25922 colonies in 20% Mueller-Hinton (control, circles) or in the presence of gold nanoparticles dispersed in Chitlac (0.2% w/v Chitlac, 1 mM HAuCl₄, Example 18) (squares). CFUs are the bacterial Colonies-Forming Units.
Figure 13. The figure shows Chitlac Raman spectrum (Example 1) at 5% w/v concentration (broken line) in comparison with Chitlac SERS spectrum with silver nanoparticles (0.4% w/v Chitlac, 14 mM AgNO₃, Example 15) (continuous line).

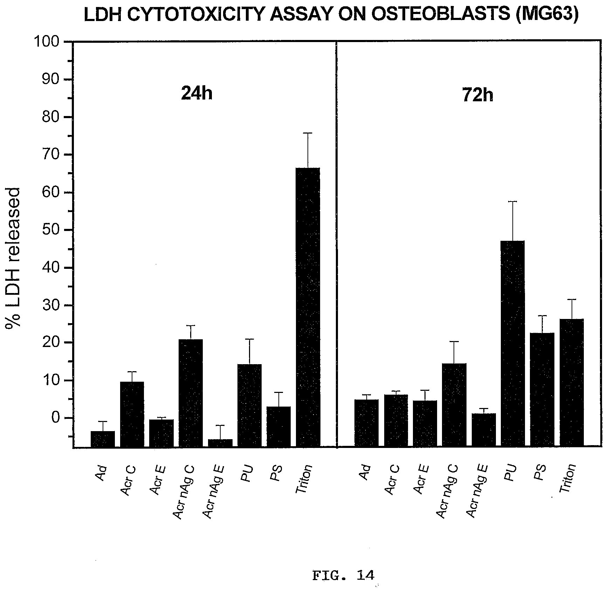

Figure 14. The figure shows the effect of coated methacrylic samples on LDH leakage from human osteosarcoma (MG63) cell lines. The cytotoxicity test was performed by direct contact of cells both with the material itself (MAcr nAg C) and with the liquid culture medium kept in contact with the material for given time (24 and 72 hours) ("extract") (MAcr nAg E). Control cells cultured in adhesion (Ad) in complete DMEM medium and cells treated with polystyrene disks (PS, negative control) and polyurethane disks with ZnDBC (PU, positive control according to ISO 10993-5) were run in parallel with the corresponding groups of methacrylic materials without nanoparticles (MAcr C, MAcr E). TritonX-100 at 0.1 % in culture medium was used as the positive control for the extracts. The percentage of LDH released was calculated by dividing the amount of activity in the medium by the total activity (medium and cell lysate) after subtraction of the control. The data are expressed as the mean (with SD range) of four independent experiments.

### Detailed description of the invention

### Definitions

*Colloidal solution (or colloid):* it means a system in which particles with size range from 1 to 1'000 nm are dispersed in a continuous solvent medium.

*Nanocomposite:* it means a system consisting of particles with nanometric sizes (*fillers*) within a macroscopic material (*matrix*). In the present description of the invention, the term "nanocomposite" is intended to mean a material in which metallic nanoparticles are dispersed in a polysaccharidic matrix. In particular it means a material in which the metallic nanoparticles are substantially formed in the polysaccharidic matrix by reduction of the corresponding salts, thus mostly have a zero charge, but without excluding the presence of *clusters* of few atoms with a ionic character (e.g. Ag₄⁺). Thus, hereinafter the nanocomposite according to the invention can be also indicated as "metal-based nanocomposite" besides simply "nanocomposite".

The objects and advantages of the nanocomposite materials comprising metallic nanoparticles interdispersed in a branched basic polysaccharide matrix object of the present invention, will be better understood throughout the following detailed description where, by way of non-limiting example, some examples of preparing nanocomposites according to the invention and the physical-chemical characterization thereof will be described in addition to the biological tests for evaluating their antibacterial activity.

### Description

In a polymeric matrix consisting of branched cationic polysaccharides due to the presence of primary and secondary nitrogen atoms, the nanocomposite materials object of the present invention comprise metallic nanoparticles homogeneously and stably dispersed in such a polymeric matrix.

For the purposes of the present invention, preferred cationic polysaccharides are alditolic or aldonic branched derivatives of chitosan, wherein D-glucosamine units forming the linear chitosan chain bind, by means of the functional group -NH- on carbon atom C2, mono- or oligo-saccharidic alditolic or aldonic polyols residues, equal to or different from each other, and represented by the previously reported general formula (**I**), where R can be -CH₂- or -CO- and R₁ can be hydrogen or a monosaccharide, preferably selected from the group consisting of galactose, glucose, mannose, N-acetyl glucosamine, and N-acetyl galactosamine, or an oligosaccharide, preferably comprising 2 glycosidic units, and R₂ can be -OH or - NHCOCH₃. Hereinafter these derivatives are also briefly referred to as alditolic or aldonic, mono- or oligo-saccharidic derivatives of chitosan or, briefly, mono- or oligo-saccharidic derivatives of chitosan.

By way of representation, D-Glucosamine units substituted with mono- or oligosaccharidic residues in the chitosan derivatives object of the invention can be represented by the general formula (**II**), where "n" refers to the overall number of D-Glucosamine units constituting a linear chitosan chain:

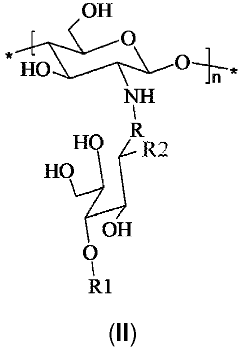

In the previously cited patent WO2007/025917A1 (Schmid H. et al.), silver nanoparticle preparations in the presence of chitosan derivatives and salts such as carboxymethylchitosan, chitosan acetate and chitosan lactate, are mentioned.

Unlike the above-cited patent, in the present case derivatization is carried out by mono- or oligosaccharidic structures which, through a reductive amination reaction, lead to the insertion of side branches linked to the polymeric backbone by means of secondary amino groups. This allows *i*) the amino groups to be still available for metal coordination and *ii*) the overall polysaccharide charge not to be substantially altered.

Indeed, the polymeric matrix consists of branched derivatives of chitosan which bind, by means of the amino group, the aldonic or alditolic derivatives of mono- or oligosaccharides of general formula (**I**). These mono- or oligo-saccharides of general formula (**I**) preferably comprise from 1 to 3 glycosidic units and, according to a more preferred aspect, these are residues of oligosaccharides comprising from 2 to 3 glycosidic units and still more preferably, with reference to the different meanings of R, R₁ and R₂, the same are selected from the group consisting of residues of lactose, cellobiose, cellotriose, maltose, maltotriose, chitobiose, chitotriose, mannobiose as well as the corresponding aldonic acids thereof. For the purposes of the present invention, the most preferred oligosaccharidic derivative of chitosan is the derivative with lactose (hereinafter also referred to as Chitlac; CAS registry number 85941-43-1).

Furthermore, for the purposes of the present invention, the chemical substitution degree of chitosan amino groups with these mono- or oligo-saccharides is at least 20%. Preferably, the substitution degree of chitosan amino groups with mono- or oligo-saccharides is in the range from 50% to 80%, and more preferably is 70%.

The average molecular weight (hereinafter referred to as MW) of the chitosan useable for obtaining the mentioned oligosaccharidic derivatives can be up to 1'500 kDa and preferably in the range from 400 kDa to 700 kDa.

The metallic nanoparticles incorporated into the polymeric matrix consisting of these branched cationic polysaccharide derivatives are of metals preferentially selected from silver, gold, platinum, palladium, copper, zinc, nickel and mixtures thereof. The nanoparticles included into the polymeric matrix consisting of oligosaccharidic derivatives of chitosan have a size in the range from 5 nm to 150 nm, and in particular a controlled average metallic nanoparticle size between 30 and 50 nm. Essential features of these nanoparticles, as will be apparent from the detailed description of the invention below, are that these nanoparticles are mostly metals in their reduced form, moreover without excluding the residual presence of clusters made of few atoms with a ionic character, and that, in their dispersion/stabilization in the polysaccharidic matrix, the oligosaccharidic side chains close to the amino groups of chitosan itself are involved.

Without being bound to the latter, the preferred ratios between matrix polysaccharides and metals are referred to nanocomposites in the form of colloidal solutions, although these metal-based nanocomposite materials can also be in the form of dehydrated films or powder and even dialyzed in order to remove residual counter-ions from the preparation of the materials themselves. In the nanocomposites according to the invention in form of aqueous colloidal solutions, the ratio of the polysaccharide concentration (expressed as % w/v) over the concentration of the starting metal salt (expressed as molarity) is from 0.0025 to 20 and preferably is 0.2.

Therefore, the silver mass, expressed as mg, which can be incorporated per gram of polysaccharide, can be from 3'000 mg/g to 0.3 mg/g and preferentially is 50 mg of silver incorporated per gram of polysaccharide.

For the pursued objects, the inventor addressed the aspect related to preparation and characterization of one polysaccharides-based system in which properties related to nanometric metal size (antimicrobial, optical, catalytic and other peculiar properties of the metals themselves) of metallic nanoparticles contained in the nanocomposites object of the invention, are exploited. These aspects are reported in detail hereinafter.

### Preparation of oligosaccharidic derivatives of chitosan

The process of preparing these oligosaccharidic derivatives of chitosan is that known and described in US 4,424,346 (Hall, L.D. and Yalpani, M.) and includes treating a solution of chitosan with acetic acid (pH 4.5) in methanol with the selected mono- or oligo-saccharide in the presence of sodium cyanoborohydride. The interaction between the amino groups of chitosan and the aldehydic group of the mono- or oligo-saccharide leads to formation of an instable intermediate referred to as Schiff's base. In the presence of borohydride, the Schiff's base is reduced thus leading to formation of a stable secondary amine. The resulting branched polysaccharide is the oligosaccharidic derivative of chitosan and is the previously mentioned Chitlac, for example, when the oligosaccharide is lactose. In case of derivatives with aldonic acids of mono- or oligo-saccharides, the reaction is carried out in the presence of an suitable activator, as a carbo-di-imide, and leads to formation of an amide.

### Preparation of nanoparticles

The nanoparticles to be incorporated into the polymeric matrix can be prepared either in the presence or absence of reducing agents.

In the first case, the nanoparticles are obtained upon reduction of metal ions with suitable reducing agents in aqueous solutions of branched polysaccharides consisting of the previously mentioned mono- or oligo-saccharidic alditolic or aldonic branched derivatives of chitosan according to the following procedure: aqueous solutions of these chitosan derivatives are prepared at different concentrations up to 2% (w/v), preferably in the range from 0.05% (w/v) to 1% (w/v) and more preferably are 0.2% (w/v). The polysaccharidic solutions are then mixed with solutions of metal salts selected from silver, gold, platinum, palladium, copper, zinc, nickel, preferably selected from chlorides, perchlorates and nitrates (e.g. AgNO₃, HAuCl₄, CuSO₄, ZnCl₂, NiCl₂), so as to obtain final concentrations in the range from 0.1 mM to 20 mM, more preferably in the range 1 mM - 14 mM and still more preferably of 1 mM. Suitable known reducing agents, preferably selected from ascorbic acid, sodium citrate, sodium borohydride and sodium cyanoborohydride, can optionally be added to the solutions to obtain nanoparticles in the metallic state. The reducing agent is added at concentrations from 0.05 mM to 10 mM and preferably the concentration is 0.5 mM.

However, it was found that unlikely for other polymeric systems, the nanocomposites according to the invention can also be prepared in the absence of reducing agents, since the mono- or oligo-saccharidic side chains according to the general formula (I) act as reducing agent for metal ions *per se,* and allow to form nanoparticles dispersed in the polymeric matrix. In this case, the metallic nanoparticles are obtainable by simply mixing the solutions of chitosan derivatives with salt solutions of the selected metal at appropriate concentrations. Also in this case, the polysaccharides and metal salt concentrations are as previously reported.

As will be readily apparent from the results obtained by the characterizations of metal-based nanocomposite materials object of the invention, hereinafter reported, all the conditions being equal, the mono- or oligo-saccharidic derivatives of chitosan, and in particular Chitlac, surprisingly prove to be more adapted to form metallic nanoparticles as compared to others polysaccharides, such as for example chitosan, as proven by the intensity and shape of the UV-Vis absorption spectra and by the spectrophotometric study assisted by transmission electron microscopy (TEM) investigations. These studies allow to demonstrate that, in the presence of the mono- or oligo-saccharidic derivatives of chitosan and in particular Chitlac, the metallic nanoparticles are more uniform in size and better dispersed due to interactions with the polymeric chains preventing the aggregation thereof in comparison with those prepared with unsubstituted chitosan. TEM microphotographs further allowed to carry out an analysis of the dimensional distribution of nanoparticles, which is highly uniform with an average size smaller than 50 nm.

It was also surprisingly found that it is possible to obtain metallic nanoparticles even in the absence of an exogenous reducing agent. A system has been discovered, in which metallic nanoparticles, which are homogeneously dispersed in the polymeric matrix, are obtained by using the reducing ability typical of the side substituents of polysaccharide, thus in the absence of any exogenous reducing agent. This second method of preparing was also characterized by means of UV-Vis spectroscopy and TEM microscopy. Plasmonic peaks and TEM images confirm the successful formation of metallic nanoparticles being roundish in shape and having an average size smaller than 10 nm.

This methodological approach is particularly advantageous because a single component of the system simultaneously offers reducing, dispersion and nanoparticle stabilization abilities.

In addition, the further biological characterizations of the metal-based nanocomposite according to the invention have shown that the nanocomposite of the invention still exhibit the antimicrobial activity of the incorporated metal and this effect is very valuable for application purposes in biomedical and pharmaceutical field. Indeed, the use of metals as silver, gold, copper, zinc and nickel in antimicrobial material field is of value, especially for the treatment of topic tissue wounds for both humans and animals. The nanocomposite object of the present could be employed as such or in combination with other diluents and/or pharmaceutically acceptable excipients. In particular, these materials can be mixed with other not-gellifying, neutral or anionic polysaccharides selected from the group consisting of dextran, hyaluronic acid, carboxymethylcellulose, galactomannans in either diluted or concentrated aqueous solution obtaining particular viscoelastic features, for preparing in combination with suitable excipients and/or diluents compositions with antimicrobial activity useable for treating infections of either intact or injured skin, mucous membranes and epithelial tissues of different anatomical localizations. These materials even in combination with other not-gellifying, neutral or anionic polysaccharides as previously mentioned can also be applied in the development of medicated devices such as gauzes, bandages, patches with broad-spectrum antimicrobial activity to be employed in the same above-mentioned therapeutic field.

Furthermore, the nanocomposites according to the invention can be used as polysaccharidic grafts (or polymer grafts) - chelating metallic nanoparticles with antimicrobial activity - on activated surfaces made of polymeric material for containers in uses in pharmaceutical and alimentary fields. The expected advantage could consist in substituting the current sterilization procedures (e.g. dry-heating) which sometimes produce even considerable lacks of homogeneity in the final sterility degree of the empty container before filling. Furthermore, coating polymeric materials can lead to obtain systems provided with antimicrobial activity for applications in the biomedical field.

Again, the metal based nanocomposite materials according to the invention can be used in polymer blends with other polysaccharides (e.g. pullulan) to obtain films for "antimicrobial" packages, in particular in the alimentary field.

A strong increase in the Raman spectroscopic signal of the polysaccharide was also found, attributable to the presence of metallic nanoparticles, which therefore have optimal dimensions and distribution to exploit SERS (Surface Enhanced Raman Spectroscopy) effect on which the operation of several molecular biosensors is based. Indeed, the Raman spectrum of a polysaccharide solution shows no experimentally detectable peak, while a polysaccharide solution (even more diluted) in the presence of metallic nanoparticles provides an intense and defined Raman spectrum, thus proving the increase of the signal caused by nanoparticles (SERS effect). In these applications, metal-based nanocomposite according to the invention can be used as probes/markers provided with molecular functionality and therefore are considered suitable for applications of optical *"bioimaging",* being the nanoparticles entrapped in the polymeric matrix formed by the branched cationic polysaccharides are alditolic or aldonic derivatives of chitosan, detectable by means of TEM microscopy, confocal and Raman techniques. Therefore, metal-based nanocomposites according to the invention can be employed in developing new sensors for analyses of biomolecules and cells by exploiting the presence of specific biochemical signals recognized by different cellular types on the oligosaccharidic derivatives of chitosan (Donati, I. et al., Biomaterials 26, 2005, 987-998).

Using branched polysaccharides to form metallic nanoparticles according to the present invention introduces the following advantages:
- the presence of amino groups on the polymeric chain allows an effective coordination of ions and, then, of metallic nanoparticles;
- the mono- or oligo-saccharidic side chains establish a steric hindrance such that they prevent metallic nanoparticles from aggregating and ensure an homogeneous and stable dispersion thereof over time;

- all conditions being equal, the branched derivatives of chitosan with aldonic or alditolic mono- or oligo-saccharides - such as for example Chitlac - were much more effective for the formation of nanoparticles as compared to chitosan in terms of dimensional homogeneity and particle dispersion (as proven by the intensity and shape of the plasmon resonance band in the UV-Vis absorption spectra in the two cases);
- in the absence of additional reducing agents, the aldonic or alditolic derivatives of mono- or oligosaccharidic side chains according to the general formula (I) act as a reducing agent for metal ions and allow to form nanoparticles dispersed in the polymeric matrix;
- the "self-reduction" ability of the branched oligosaccharidic derivative of chitosan - metal nanoparticles complex allows to reduce the number of chemical components which form the system;
- preparation simplicity and reproducibility of the results;
- wide applications of nanocomposites for both their broad-spectrum antimicrobial activity and the peculiar optical properties in applications of Raman spectroscopy (SERS);
- possible use in polymer blends with other polysaccharides (e.g. pullulan) to obtain films for "antimicrobial" packages, in particular in the alimentary field.

Some non-limiting examples of preparing the nanocomposites according to the invention, on which the physical-chemical characterizations and biological tests were performed to evaluate their antibacterial activity are described hereinafter for illustrative purpose.

### Example 1: synthesis of chitosan derivative with lactose (Chitlac)(1a) and chitosan derivative with cellobiose (Chitcell) (1b)

*1a)* Chitosan (1.5 g, acetylation degree 11%) is dissolved into 110 mL of a methanol solution (55 mL) and 1% w/v acetic acid buffer, pH 4.5 (55 mL). 60 mL of a methanol solution (30 mL) and 1% w/v acetic acid buffer, pH 4.5 (30 mL) containing lactose (2.2 g) and sodium cyanoborohydride (900mg) are added. The mixture is left to stir for 24 hours, transferred to dialysis tubes (cut off 12'000Da) and dialyzed against NaCl 0.1 M (2 changes) and against deionized water until a conductivity of 4µS at 4°C is achieved. Ultimately, the solution is filtered on Millipore 0.45µm filters and freeze-dried.
*1b)* Chitosan (1.5 g, acetylation degree 11%) is dissolved in 110 mL of a methanol solution (55 mL) and 1% w/v acetic acid buffer, pH 4.5 (55 mL). 60 mL of a methanol solution (30 mL) and 1% w/v acetic acid buffer, pH 4.5 (30 mL) containing cellobiose (2.2 g) and sodium cyanoborohydride (900 mg) are added. The mixture is left to stir for 24 hours, transferred to dialysis tubes (cut off 12'000Da) and dialyzed against NaCl 0.1 M (2 changes) and against deionized water until a conductivity of 4µS at 4°C is achieved. Ultimately, the solution is filtered on Millipore 0.45µm filters and freeze-dried.

### Example 2: Preparation of silver nanoparticles in Chitlac in the presence of reducing agents

Nanoparticles are obtained upon reduction of metal ions with ascorbic acid in Chitlac solution is according to the following procedure: an aqueous Chitlac solution at a concentration of 0.05 % (w/v) is prepared. The Chitlac solution is then mixed with silver nitrate solutions so as to obtain a final concentration of AgNO₃ of 0.5 mM. Then, a solution of ascorbic acid is added so as to obtain a final concentration of 0.25 mM.

### Example 3: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.05 % (w/v) is prepared. The Chitlac solutions then mixed with silver nitrate solutions so as to obtain a final concentration of AgNO₃ of 1 mM. Then, a solution of ascorbic acid is added so as to obtain a final concentration of 0.5 mM.

### Example 4: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.1 % (w/v) so as order to obtain a final concentration of AgNO₃ of 0.5 mM. Then, a solution of ascorbic acid is added so as to obtain a final concentration of 0.25 mM.

### Example 5: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.1 % (w/v) is prepared. The Chitlac solution is then mixed with silver nitrate solutions so as to obtain a final concentration of AgNO₃ of 1 mM. Then, a solution of ascorbic acid is added so as to obtain a final concentration of 0.5 mM.

### Example 6: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.2 % (w/v) is prepared. The Chitlac solution is then mixed with silver nitrate solutions so as to obtain a final concentration of AgNO₃ of 0.5 mM. Then, a solution of ascorbic acid is added so as to obtain a final concentration of 0.25 mM.

### Example 7: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.2 % (w/v) is prepared. The Chitlac solutions are then mixed with silver nitrate solutions so as to obtain a final concentration of AgNO₃ of 1 mM. Then, a solution of ascorbic acid is added so as to obtain a final concentration of 0.5 mM.

### Example 8: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.4 % (w/v) is prepared. The Chitlac solutions are then mixed with silver nitrate solutions so as to obtain a final concentration of AgNO₃ of 0.5 mM. Then, a solution of ascorbic acid is added so as to obtain a final concentration of 0.25 mM.

### Example 9: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 0.4 % (w/v) is prepared. The Chitlac solutions are then mixed with silver nitrate solutions so as to obtain a final concentration of AgNO₃ of 1 mM. Then, a solution of ascorbic acid is added so as to obtain a final concentration of 0.5 mM.

### Example 10: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.2 % (w/v) is prepared. The Chitlac solution is then mixed with a silver nitrate solution so as to obtain a final concentration of AgNO₃ of 5 mM.

### Example 11: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.2 % (w/v) is prepared. The Chitlac solution is then mixed with a silver nitrate solution so as to obtain a final concentration of AgNO₃ of 10 mM.

### Example 12: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.2 % (w/v) is prepared. The Chitlac solution is then mixed with a silver nitrate solution so as to obtain a final concentration of AgNO₃ of 14 mM.

### Example 13: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at concentration of 0.4 % (w/v) is prepared. The Chitlac solution is then mixed with a silver nitrate solution so as to obtain a final concentration of AgNO₃ of 5 mM.

### Example 14: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.4 % (w/v) is prepared. The Chitlac solution is then mixed with a silver nitrate solution so as to obtain a final concentration of AgNO₃ of 10 mM.

### Example 15: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.4 % (w/v) is prepared. The Chitlac solution is then mixed with a silver nitrate solution so as to obtain a final concentration of AgNO₃ of 14 mM.

### Example 16: Preparation of gold nanoparticles in Chitlac in the presence of reducing agents

Nanocomposites comprising the nanoparticles are obtained upon reduction of metal ions with sodium borohydride in Chitlac solutions according to the following procedure: an aqueous Chitlac solution at a concentration of 0.2 % (w/v) is prepared. The Chitlac solutions are then mixed with tetrachloroauric acid solutions so as to obtain a final concentration of HAuCl₄ of 1 mM. Then, a solution of sodium borohydride is added so as to obtain a final concentration of 0.3 mM.

### Example 17: Preparation of gold nanoparticles in Chitlac in the presence of reducing agents

Nanocomposites comprising the nanoparticles are obtained upon reduction of metal ions with sodium borohydride in Chitlac solutions according to the following procedure: an aqueous Chitlac solution at a concentration of 0.4 % (w/v) is prepared. The Chitlac solutions are then mixed with tetrachloroauric acid solutions so as to obtain a final concentration of HAuCl₄ of 1 mM. Then, a solution of sodium borohydride is added so as to obtain a final concentration of 0.3 mM.

### Example 18: Preparation of gold nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.2 % (w/v) is prepared. The Chitlac solution is then mixed with a tetrachloroauric acid solution so as to obtain a final concentration of HAuCl₄ of 1 mM.

### Example 19: Preparation of copper nanoparticles in Chitlac in the presence of reducing agents

Nanocomposites comprising the nanoparticles are obtained upon reduction of metal ions with sodium borohydride in Chitlac solutions according to the following procedure: an aqueous Chitlac solution at a concentration of 0.2 % (w/v) is prepared. The Chitlac solutions are then mixed with copper sulphate solutions so as to obtain a final concentration of CuSO₄ of 1 mM. Then, a solution of sodium borohydride is added so as to obtain a final concentration of 0.2 mM.

### Example 20: Preparation of silver nanoparticles in Chitcell (cellobiose derivative of chitosan) in the absence of reducing agents

Nanocomposites comprising the nanoparticles are obtained upon reduction of metal ions by the polysaccharide Chitcell according to the following procedure: an aqueous Chitcell solution at a concentration of 0.4 % (w/v) is prepared. The Chitcell solution is then mixed with a silver nitrate solution so as to obtain a final concentration of AgNO₃ of 14 mM.

### Example 21: Preparation of silver nanoparticles in Chitgluc (glucose derivative of chitosan) in the absence of reducing agents

Nanocomposites comprising the nanoparticles are obtained upon reduction of metal ions by the polysaccharide Chitgluc according to the following procedure: an aqueous Chitgluc solution at a concentration of 0.4 % (w/v) is prepared. The Chitgluc solution is then mixed with a silver nitrate solution so as to obtain a final concentration of AgNO₃ of 14 mM.

### Example 22: Preparation of silver nanoparticles in Chitosan in the presence of reducing agents

Nanocomposites comprising the nanoparticles are obtained upon reduction of metal ions with ascorbic acid in Chitosan solutions according to the following procedure: an aqueous Chitosan solution at a concentration of 0.2 % (w/v) is prepared. The Chitosan solutions are then mixed with silver nitrate solutions so as to obtain a final concentration of AgNO₃ of 1 mM. Then, a solution of ascorbic acid is added so as to obtain a final concentration of 0.5 mM.

### Example 23: Preparation of silver nanoparticles in Chitlac in the presence of reducing agents

Nanocomposites comprising the nanoparticles are obtained upon reduction of metal ions with ascorbic acid in Chitlac solutions according to the following procedure: an aqueous Chitlac solution at a concentration of 1 % (w/v) is prepared. The Chitlac solutions are then mixed with silver nitrate solutions so as to obtain a final concentration of AgNO₃ of 1 mM. Then, a solution of ascorbic acid is added so as to obtain a final concentration of 0.5 mM.

### Example 24: Preparation of silver nanoparticles in Chitlac in the presence of reducing agents

Nanoparticles are obtained upon reduction of metal ions with ascorbic acid in Chitlac solutions according to the following procedure: an aqueous Chitlac solution at a concentration of 2 % (w/v) is prepared. The Chitlac solutions are then mixed with silver nitrate solutions so as to obtain a final concentration of AgNO₃ of 1 mM. Then, a solution of ascorbic acid is added so as to obtain a final concentration of 0.5 mM.

### Example 25: Preparation of silver nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.2 % (w/v) is prepared. The Chitlac solution is then mixed with a silver nitrate solution so as to obtain a final concentration of AgNO₃ of 1 mM.

### Example 26: Preparation of gold nanoparticles in Chitlac in the absence of reducing agents

Nanocomposites comprising the nanoparticles are obtained upon reduction of metal ions by the polysaccharide Chitlac according to the following procedure: an aqueous Chitlac solution at a concentration of 0.4 % (w/v) is prepared. The Chitlac solution is then mixed with a tetrachloroauric acid solution so as to obtain a final concentration of HAuCl₄ of 1 mM.

The physical-chemical and biological characterizations of metal-based nanocomposites according to the invention obtained with the chitosan derivative with lactose, Chitlac, with nanoparticles of different metals, and in particular Ag and Au are reported below. Similar results were obtained with all oligosaccharidic derivatives object of the present invention.

### Nanoparticle stabilization

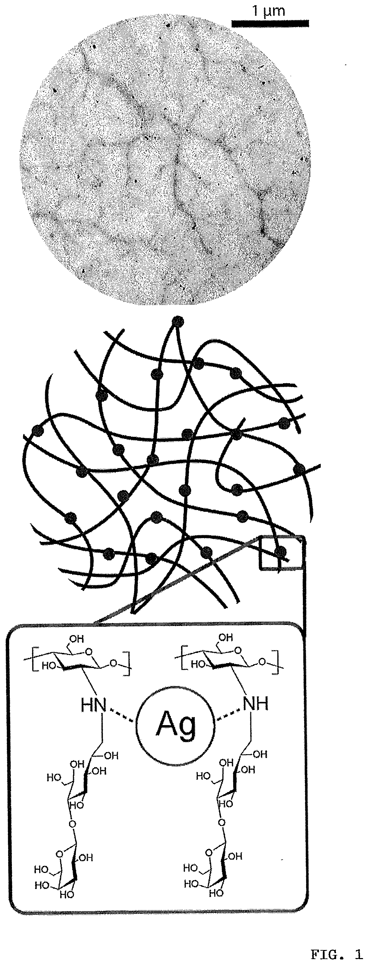

In order to check the formation of nanoparticles and their stabilization in the polymeric matrix according to the objects of the invention, the nanocomposite of example 7 was subjected to transmission electron microscopy. As underlined in figure 1, the presence of several functional groups (amino groups, hydroxyl groups) promotes coordinating interactions with metal ions, while the presence of the side chains of mono or oligo-saccharides, such as for example lactose, offers an effective steric hindrance so as to hamper the natural tendency of nanoparticles to aggregate. Indeed, when a solution of AgNO₃ is added to a Chitlac solution, coordinating interactions between Ag⁺ ions and amino groups are established; upon reduction, the side chains of lactose close to the amino groups are involved in metallic nanoparticle stabilization.

### UV-Vis characterization

The nanoparticle formation is evidenced by the appearance of absorption bands at wavelengths which vary depending on the considered metal, in UV-Vis absorption spectra; these bands are known under the name of surface plasmon resonance bands and are caused by a collective oscillation of free electrons on the nanoparticle surface. In the case of silver, for example, an intense band centered at about 400 nm is observed (Figure 2A); the plasmon band being symmetrical and very narrow suggests that well dispersed, predominantly sphere-shaped nanoparticles are present. The comparison of absorption spectra of nanoparticles formed in Chitlac and in chitosan, all conditions being equal, underlines the different behavior of the two polysaccharides. Indeed, in case of Chitlac the plasmon band is much more intense, symmetrical, narrow and shifted towards lower wavelengths, which suggests the presence of smaller and better dispersed particles (Figure 2A). The better properties of Chitlac as compared to chitosan can be ascribed to physical-chemical properties of the side chains of lactose placed on the polymeric chain. The system is adjustable depending on the concentrations used, as proven by the spectrophotometric characterizations made (Figure 2B).

The plasmonic resonance band is particularly significant and diagnostic.

Spectra further confirm that metal particles included in the polymeric matrix mostly have a zero charge; however clusters of few atoms with a ionic character can be present (e.g. Ag₄⁺), as indicated by the shoulder at about 260 nm in the UV absorption spectrum of the systems (Figure 2, A and B).

The study of UV-Vis spectrum variations according to the concentrations and reaction times allowed to improve an optimal method of preparing in terms of yield, reproducibility and stability over time.

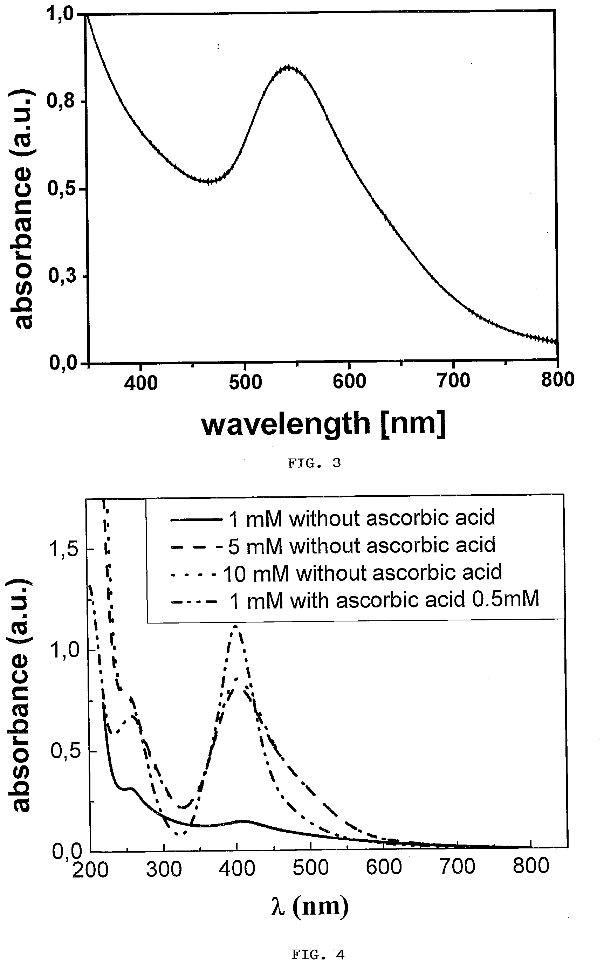

Similarly, in case of gold, an intense plasmonic band around 530 nm, which proves the successful formation of gold nanoparticles dispersed in Chitlac (Figure 3) is detected.

### Reducing properties of Chitlac

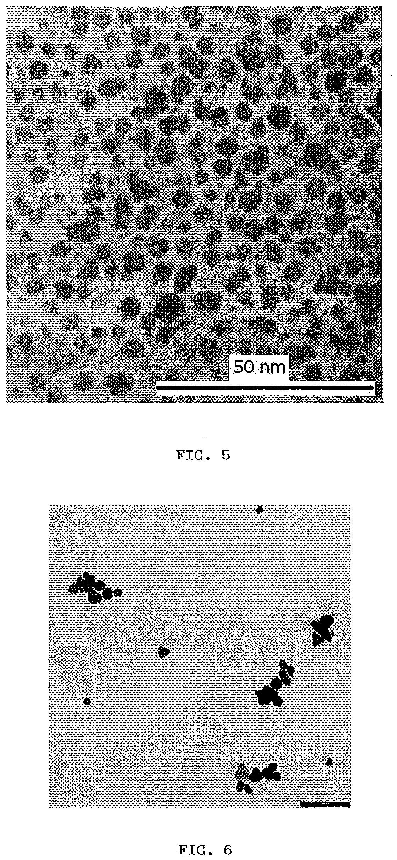

Surprisingly, it was found that, under appropriate conditions, metallic nanoparticles can be formed even in the absence of added reducing agents; for example, in the case of silver, Chitlac is able to reduce Ag⁺ ions without the aid of ascorbic acid, for example. UV-Vis spectra (Figure 4) and TEM images (Figure 5) confirm the successful formation of stable and well-dispersed metallic nanoparticles. It should further note that, under appropriate conditions, the system based on Chitlac and silver nanoparticles, free of reducing agents, is capable of forming hydrogels without adding further chemical agents. Also, these gels get looser and eventually melt over time (about 1 week).

Even in the case of gold, Chitlac free of added reducing agents allows the reduction of Au³⁺ ions to the metallic state, as proven by UV-Vis spectra in Figure 3, where the plasmonic peak of gold is observed, and by TEM images in Figure 6 where nanoparticles with average size around 50 nm are observed.

### TEM characterization and dimensional analysis:

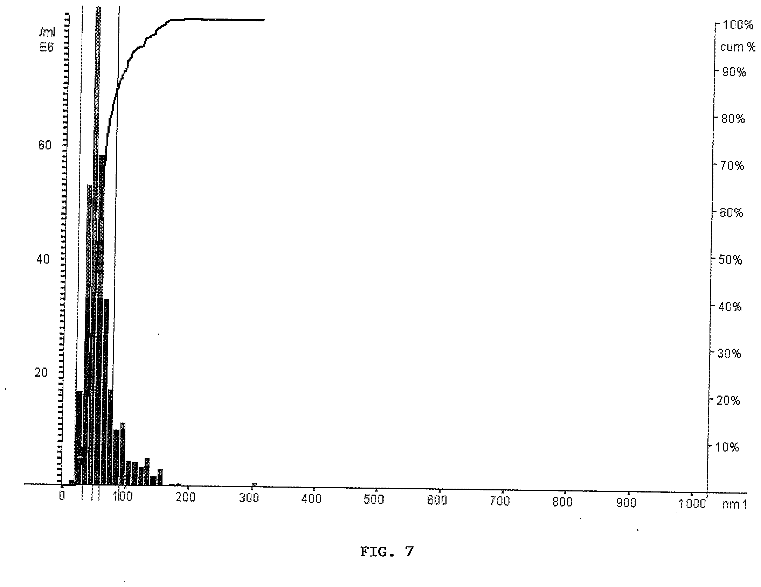

The shape, distribution and dimensions of nanoparticles formed in the presence and absence of reducing agents were evaluated by means of Transmission electron microscopy (TEM), Nanoparticle Tracking Analysis (Nanosight®) and image analysis techniques. The images related to gold nanoparticles (Figures 6 and 7) show the presence of nanoparticles with an average sizes around 50 nm.

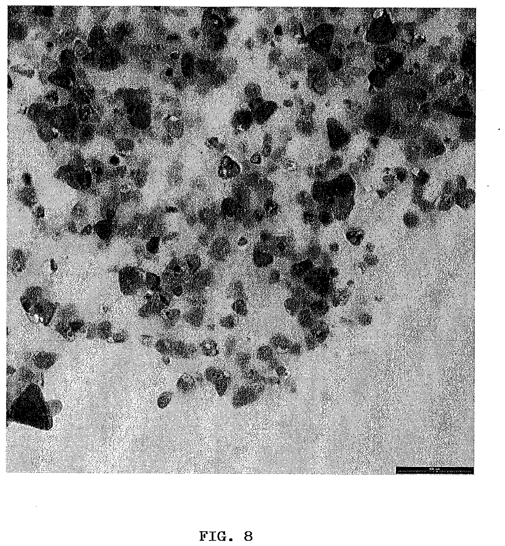

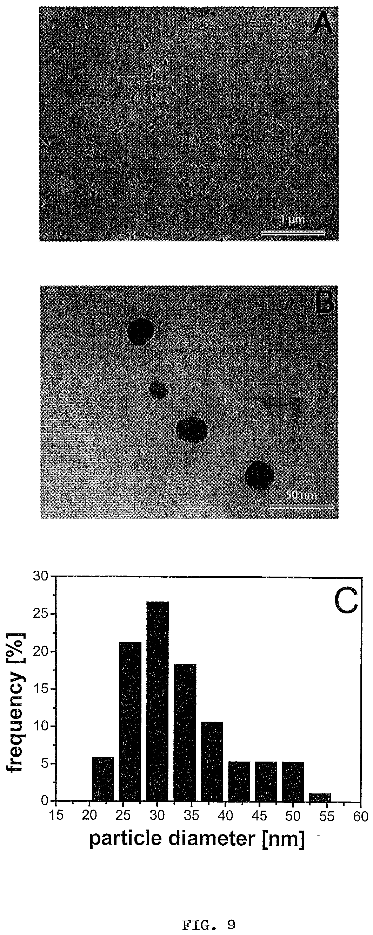

Even in the case of copper, the successful formation of metallic nanoparticles is clearly observed (Figure 8). In Figure 9, TEM images of silver nanoparticles being roundish in shape and having average dimensions around 30 nm are shown.

This method of preparing without reducing agents was also characterized by means of UV-Vis spectroscopy and TEM microscopy. Plasmonic peaks in Figure 4 and TEM images in Figure 5 confirm the successful formation of metallic nanoparticles being roundish in shape and having an average size smaller than 10 nm.

In order to evaluate the antimicrobial activity, nanocomposite systems were tested with different Gram+ and Gram- bacterial strains; growth kinetic tests and counting tests on bacterial colonies have demonstrated a strong and rapid bactericidal effect.

### Antibacterial activity:

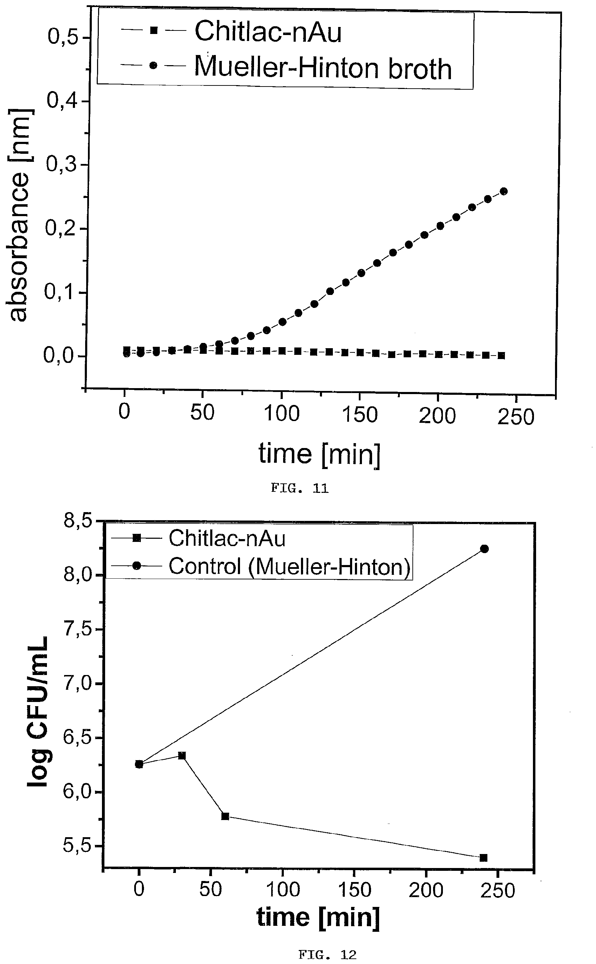

Growth inhibition tests were performed on different bacterial strains (Gram negative: *Escherichia coli, Pseudomonas aeruginosa,* and Gram positive: *Staphylococcus aureus, Staphylococcus epidermidis*) in the presence of Chitlac solutions with and without metallic nanoparticles. It was found that bacterial growth in Chitlac solutions free from nanoparticles is comparable to the growth in control (Mueller-Hinton broth); in contrast, a considerable bacterial growth inhibition occurs in the presence of Chitlac with metallic nanoparticles, as reported for example in the case of silver and gold (Figures 10, 11 and 12) in which the decrease of the number of bacterial colonies according to the incubation time may be seen. This process is highly fast: even after a few hours an effective inhibition of bacterial cells is indeed found.

### Raman spectroscopic characterization

Raman spectra on solutions containing polymer-stabilized nanoparticles were collected using an *inVia* Raman system (Renishaw plc, Wotton-under-Edge, U.K.). The laser (514.5 nm argon-ion laser, LaserPhysics, West Jordan UT, U.S.A.) was focused on the sample by a 20x objective (0.4 NA). Laser power at the sample was 3 mW; to minimize laser-induced photodegradation of the sample, the laser power density was lowered by defocusing the beam by 10%. The exposure time was 5 accumulations of 10 s for each spectrum, for a total exposure time of 50 s per spectrum.

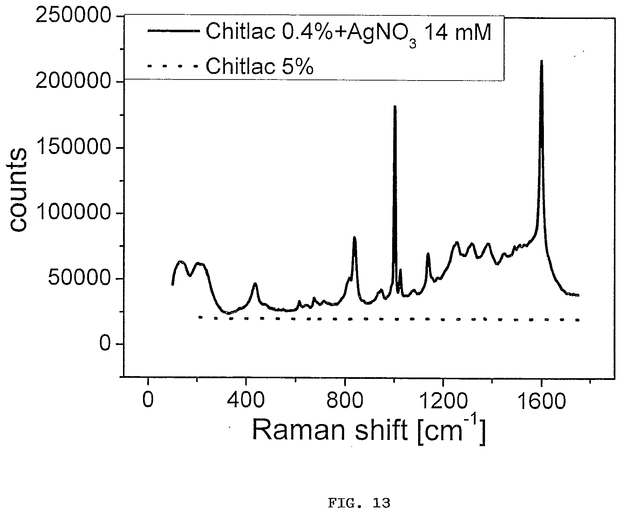

A strong increase in the Raman spectroscopic signal of the polysaccharide was found, which can be ascribed to the presence of metallic nanoparticles. The latter ones therefore have optimal dimensions and size distribution to exploit the SERS (Surface Enhanced Raman Spectroscopy) effect on which the operation of several molecular biosensors is based. Indeed, as inferred from figure 13, Raman spectrum of a polysaccharide solution shows no detectable peaks, while an even more diluted solution of the same polysaccharide in the presence of metallic nanoparticles provides an intense and defined Raman spectrum, thus proving the increase of the signal caused by nanoparticles (SERS effect).

### Application of the metal-based nanocomposite for coating methacrylic resins

Methacrylic resins cylindrical samples (Ø=1,4mm; h=2mm) were immersed in HCl 12 M for 7 hours at 80°C; then the samples were rinsed with deionized water (2X 50mL), NaOH 0.1 M (50 mL) and water again (50mL). The acid-treated methacrylic resin samples were immersed in the colloidal solutions of the metal-based nanocomposite prepared according to example 9 for 24 hours. Finally the samples were rinsed in deionized water and dried under hood.

Some biological characterization of the coated methacrylic resins are hereinafter reported.

### Antimicrobial efficacy assay

Cylindrical samples (Ø=1,4mm; h=2mm) of coated methacrylic resins were put in direct contact with *S. aureus* bacteria. On each sample 10µL of bacteria suspension were plated at the concentration of 10⁶ CFU/mL (CFU=Colony Forming Units) and incubated in a moist environment at 37°C for 3 hours. Then, each sample was immersed in 2mL of PBS in agitation to allow the bacteria detachment from the surface. These washing solutions were serially diluted and plated on agar gel to allow colony count after overnight incubation at 37°C. The colony count numbers demonstrate that in the case of methacrylic resins coated with Chitlac-nAg the number of bacteria on the surface is reduced by 98%.

### LDH cytotoxicity assay

After the antibacterial tests, further investigations were carried out, aimed at evaluating the toxicity of the nanocomposite coating towards eukaryotic cells. To this end, LDH cytotoxicity assays were performed by direct contact of osteoblasts-like cells (MG63) both with the material itself and with the liquid culture medium kept in contact with the material for given time (24 hours) ("extract"). Tested materials (in quadruplicate) were directly deposited on the cell layer. After 24 and 72 h, the medium was collected and the LDH assay was performed according to the manufacture's protocol. The results reported in figure 14 show that after 72 hours there is no evidence of cytotoxicity associated with the material.

## Claims

1. Nanocomposite materials comprising a polymeric matrix consisting of cationic polysaccharides selected from branched derivatives of chitosan, wherein the D-glucosamine units forming the chitosan linear chain bind, by means of the functional group -NH- on carbon atom C2, the alditolic or aldonic polyols residues, equal or different from each other, represented by the general formula (II): 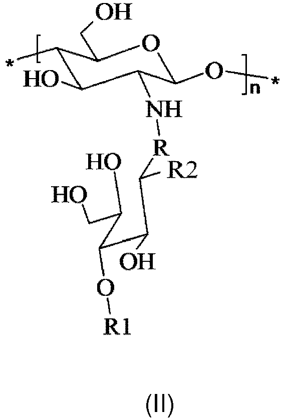 wherein:
- R is -CH₂- or -CO-;
- R₁ is hydrogen, a monosaccharide, an oligosaccharide;
- R₂ is -OH or -NHCOCH₃
and metallic nanoparticles formed on the polysaccharide matrix by reduction of the corresponding salts of said metals and homogeneously dispersed and stabilized in said polymeric matrix.

2. Nanocomposite materials according to claim 1, wherein said nanoparticles are nanoparticles of metals selected from the group consisting of silver, gold, platinum, copper, zinc, nickel and mixtures thereof.

3. Nanocomposite materials according claim 1, wherein the alditolic or aldonic polyols residues are residues of mono-or oligosaccharides comprising from 1 to 3 glycosidic units.

4. Nanocomposite materials according to claim 1, wherein, when R₁ is a monosaccharide, said monosaccharide is selected from the group consisting of galactose, glucose, mannose, N-acetyl glucosamine, and N-acetyl galactosamine.

5. Nanocomposite materials according claim 1, wherein the alditolic or aldonic polyols residues are selected from the group consisting of residues of lactose, cellobiose, cellotriose, maltose, maltotriose, chitobiose, chitotriose, mannobiose and aldonic acids thereof.

6. Nanocomposite materials according to claim 1, wherein the chitosan branched derivatives have a substitution degree of the D-Glucosamine unit amine group higher than 20% and up to 80%.

7. Nanocomposite materials according to claim 1, wherein the nanoparticles have an average size comprised from 5 to 150 nm.

8. Nanocomposite materials according to claim 1, wherein the metal mass in mg included into the polysaccharidic matrix in g of polysaccharide is comprised from 3'000 mg/g to 0.3 mg/g.

9. Nanocomposite materials according to one of claims 1-8 for use as antimicrobial agents.

10. Nanocomposite materials according to claim 9, wherein said nanocomposite materials are used in combination with pharmaceutically acceptable excipients and/or diluents, for treatment of infections of skin, mucous membranes and epithelial tissues, intact or wounded of different anatomical sites.

11. Nanocomposite materials according to claim 10, wherein said nanocomposite materials are in a mixture with neutral or anionic not-gellifying anionic polysaccharides selected in the group consisting of dextran, hyaluronic acid, carboxymethylcellulose and galactomannans.

12. Nanocomposite materials according to one of claims 1-8 for use in applications of SERS (*Surface Enhanced Raman Spectroscopy*).

13. Nanocomposite materials according to to one of claims 1-8 for use in molecular biosensors.

14. Nanocomposite materials according to one of claims 1-8 for use as polysaccharidic coatings on activated surfaces of polymeric materials.

15. Nanocomposite materials according to one of claims 1-8 for use in mixtures with other polysaccharides for preparing packaging films.

16. Method of preparation of nanocomposite materials according to one of the claims 1-8 comprising the at least the steps of:
a) preparing aqueous solutions of branched cationic polysaccharides in a concentration up to 2% (w/v);
b) preparing aqueous solutions of metal salts in a concentration comprised from 0.1 mM to 14mM;
c) adding said metal salts solutions to the polysaccharide solutions and mixing up to reduction of the metal ions and formation of colloidal solutions of metal nanoparticles wherein said polysaccharide-reduced metallic nanoparticles are homogeneously dispersed.

17. Method of preparation according to claim 16 further comprising the addition of an aqueous solution of an exogenous reducing agent in a concentration comprised from 0.05 mM to 10 mM.

18. Method of preparation according to claim 16, wherein the polysaccharide concentrations are comprised from 0.05 (w/v) to 1% (w/v) and the salt concentrations are comprised from 1 mM to 14 mM.

19. Devices comprising nanocomposite materials according to one of claims from 1 to 8 for use in the treatment of infections of skin, mucous membranes and epithelial tissues, intact or wounded, of different anatomical sites.

## Patentansprüche

1. Nanoverbundstoffe, umfassend eine Polymermatrix, bestehend aus kationischen Polysacchariden, ausgewählt aus verzweigten Derivaten von Chitosan, wobei die lineare Chitosankette bildenden D-Glucosamineinheiten mittels der funktionellen Gruppe -NH- an Kohlenstoff C2 Alditol- oder Aldonpolyolreste binden, die gleich oder verschieden voneinander sind, dargestellt durch die allgemeine Formel (II): 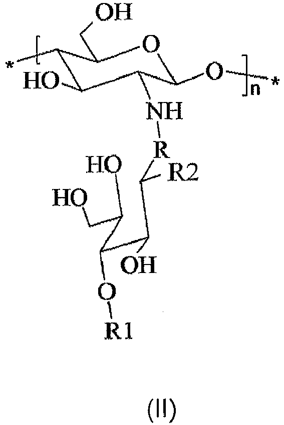 wobei:
- R -CH₂- oder -CO- ist;
- R₁ Wasserstoff, ein Monosaccharid, ein Oligosaccharid ist;
- R₂ -OH oder -NHCOCH₃ ist
und Metallnanopartikel, die auf der Polysaccharidmatrix durch Reduktion der entsprechenden Salze der Metalle gebildet sind und in der Polymermatrix homogen verteilt und stabilisiert sind.

2. Nanoverbundstoffe nach Anspruch 1, wobei die Nanopartikel Nanopartikel von Metallen sind, ausgewählt aus der Gruppe, bestehend aus Silber, Gold, Platin, Kupfer, Zink, Nickel und deren Mischungen.

3. Nanoverbundstoffe nach Anspruch 1, wobei die Alditol- oder Aldonpolyolreste Reste von Mono- oder Oligosacchariden sind, die 1 bis 3 Glycosideinheiten umfassen.

4. Nanoverbundstoffe nach Anspruch 1, wobei, wenn R₁ ein Monosaccharid ist, das Monosaccharid ausgewählt ist aus der Gruppe, bestehend aus Galactose, Glucose, Mannose, N-Acetylglucosamin und N-Acetylgalactosamin.

5. Nanoverbundstoffe nach Anspruch 1, wobei die Alditol- oder Aldonpolyolreste aus der Gruppe ausgewählt sind, bestehend aus Resten von Laktose, Cellobiose, Cellotriose, Maltose, Maltotriose, Chitobiose, Chitotriose, Mannobiose und deren Aldonsäuren.

6. Nanoverbundstoffe nach Anspruch 1, wobei die verzweigten Chitosanderivate einen Substitutionsgrad der Amingruppen der D-Glucosamineinheiten von höher als 20 % und bis zu 80 % aufweisen.

7. Nanoverbundstoffe nach Anspruch 1, wobei die Nanopartikel eine durchschnittliche Größe zwischen 5 und 150 nm aufweisen.

8. Nanoverbundstoffe nach Anspruch 1, wobei die Metallmasse in mg, die in der Polysaccharidmatrix enthalten ist, in g von Polysaccharid 3000 mg/g bis 0,3 mg/g beträgt.

9. Nanoverbundstoffe nach einem der Ansprüche 1 bis 8 zur Verwendung als antimikrobielle Wirkstoffe.

10. Nanoverbundstoffe nach Anspruch 9, wobei die Nanoverbundstoffe in Kombination mit pharmazeutisch akzeptablen Vehikeln und/oder Verdünnungsmitteln zur Behandlung von Infektionen der Haut, Schleimhautmembranen und Epithelgeweben, intakt oder verwundet, von unterschiedlichen anatomischen Stellen verwendet werden.

11. Nanoverbundstoffe nach Anspruch 10, wobei die Nanoverbundstoffe in einer Mischung mit neutralen oder anionischen, nicht gelifizierenden anionischen Polysacchariden vorliegen, ausgewählt aus der Gruppe, bestehend aus Dextran, Hyaluronsäure, Carboxymethylcellulose und Galactomannane.

12. Nanoverbundstoffe nach einem der Ansprüche 1 bis 8 zur Verwendung in Anwendungen von SERS (*Surface Enhanced Raman Spectroscopy*).

13. Nanoverbundstoffe nach einem der Ansprüche 1 bis 8 zur Verwendung bei molekularen Biosensoren.

14. Nanoverbundstoffe nach einem der Ansprüche 1 bis 8 zur Verwendung als Polysaccharidbeschichtungen auf aktivierten Oberflächen von Polymermaterialien.

15. Nanoverbundstoffe nach einem der Ansprüche 1 bis 8 zur Verwendung bei Mischungen mit anderen Polysacchariden zur Herstellung von Verpackungsfolien.

16. Verfahren zur Herstellung von Nanoverbundstoffen nach einem der Ansprüche 1 bis 8, umfassend zumindest die Schritte:
a) Herstellen wässriger Lösungen von verzweigten kationischen Polysacchariden in einer Konzentration von bis zu 2 % (w/v);
b) Herstellen wässriger Lösungen von Metallsalzen in einer Konzentration, die von 0,1 mM bis 14 mM umfasst;
c) Zugeben der Metallsalzlösungen zu den Polysaccharidlösungen und Vermischen zur Reduktion der Metallionen und Bildung kolloidaler Lösungen von Metallnanopartikeln, wobei die polysaccharidreduzierten Metallnanopartikel homogen verteilt werden.

17. Verfahren zur Herstellung nach Anspruch 16, das ferner die Zugabe einer wässrigen Lösung von einem exogenen Reduziermittel in einer Konzentration, die 0,05 mM bis 10 mM beträgt, umfasst.

18. Verfahren zur Herstellung nach Anspruch 16, wobei die Polysaccharidkonzentrationen von 0,05 (w/v) bis 1 % (w/v) umfassen, und die Salzkonzentrationen 1 mM bis 14 mM betragen.

19. Vorrichtungen, die Nanoverbundstoffe nach einem der Ansprüche 1 bis 8 umfassen, zur Verwendung bei der Behandlung von Infektionen der Haut, Schleimhautmembranen und Epithelgeweben, intakt oder verwundet, von unterschiedlichen anatomischen Stellen.

## Revendications

1. Matériaux nanocomposites comprenant une matrice polymère constituée de polysaccharides cationiques sélectionnés parmi des dérivés ramifiés de chitosane, où les unités D-glucosamine formant la chaîne linéaire du chitosane se lient, au moyen du groupe fonctionnel -NH- sur l'atome de carbone C2, aux résidus de polyols alditoliques ou aldoniques, identiques ou différents les uns des autres, représentés par la formule générale (II) : 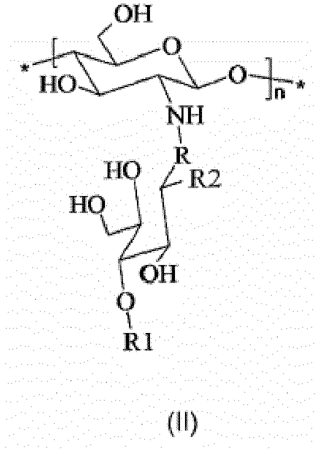 où :
- R est -CH₂- ou -CO- ;
- R₁ est un hydrogène, un monosaccharide, un oligosaccharide ;
- R₂ est -OH ou -NHCOCH₃
et des nanoparticules métalliques formées sur la matrice de polysaccharide par réduction des sels correspondants desdits métaux et dispersées de manière homogène et stabilisées dans ladite matrice polymère.

2. Matériaux nanocomposites selon la revendication 1, dans lesquels lesdites nanoparticules sont des nanoparticules de métaux sélectionnés dans le groupe constitué par l'argent, l'or, le platine, le cuivre, le zinc, le nickel et leurs mélanges.

3. Matériaux nanocomposites selon la revendication 1, dans lesquels les résidus de polyols alditoliques ou aldoniques sont des résidus de mono- ou d'oligosaccharides comprenant de 1 à 3 unités glycosidiques.

4. Matériaux nanocomposites selon la revendication 1, dans lesquels, lorsque R₁ est un monosaccharide, ledit monosaccharide est sélectionné dans le groupe constitué par le galactose, le glucose, le mannose, la N-acétylglucosamine, et la N-acétylgalactosamine.

5. Matériaux nanocomposites selon la revendication 1, dans lesquels les résidus de polyols alditoliques ou aldoniques sont sélectionnés dans le groupe constitué par les résidus de lactose, cellobiose, cellotriose, maltose, maltotriose, chitobiose, chitotriose, mannobiose et leurs acides aldoniques.

6. Matériaux nanocomposites selon la revendication 1, dans lesquels les dérivés ramifiés de chitosane ont un degré de substitution du groupe amine de l'unité D-glucosamine supérieur à 20 % et jusqu'à 80 %.

7. Matériaux nanocomposites selon la revendication 1, dans lesquels les nanoparticules ont une taille moyenne comprise entre 5 et 150 nm.

8. Matériaux nanocomposites selon la revendication 1, dans lesquels la masse de métal en mg incluse dans la matrice de polysaccharide en g de polysaccharide est comprise entre 3000 mg/g et 0,3 mg/g.

9. Matériaux nanocomposites selon l'une des revendications 1 à 8 destinés à être utilisés en tant qu'agents antimicrobiens.

10. Matériaux nanocomposites selon la revendication 9, lesdits matériaux nanocomposites étant utilisés en combinaison avec des excipients et/ou diluants pharmaceutiquement acceptables, pour le traitement d'infections de la peau, des muqueuses et des tissus épithéliaux, intacts ou lésés, de différents sites anatomiques.

11. Matériaux nanocomposites selon la revendication 10, lesdits matériaux nanocomposites étant dans un mélange avec des polysaccharides anioniques non gélifiants neutres ou anioniques sélectionnés dans le groupe constitué par le dextrane, l'acide hyaluronique, la carboxyméthylcellulose et les galactomannanes.

12. Matériaux nanocomposites selon l'une des revendications 1 à 8 destinés à être utilisés dans des applications de SERS (*Surface Enhanced Raman Spectroscopy,* ou spectroscopie Raman exaltée par effet de surface).

13. Matériaux nanocomposites selon l'une des revendications 1 à 8 destinés à être utilisés dans des biocapteurs moléculaires.

14. Matériaux nanocomposites selon l'une des revendications 1 à 8 destinés à être utilisés en tant que revêtements polysaccharidiques sur des surfaces activées de matériaux polymères.

15. Matériaux nanocomposites selon l'une des revendications 1 à 8 destinés à être utilisés dans des mélanges avec d'autres polysaccharides pour la préparation de films d'emballage.

16. Procédé de préparation de matériaux nanocomposites selon l'une des revendications 1 à 8 comprenant au moins les étapes consistant à :
a) préparer des solutions aqueuses de polysaccharides cationiques ramifiés, à une concentration jusqu'à 2 % (p/v) ;
b) préparer des solutions aqueuses de sels métalliques à une concentration comprise entre 0,1 mM et 14 mM ;
c) ajouter lesdites solutions de sels métalliques aux solutions de polysaccharides et mélanger jusqu'à la réduction des ions métalliques et la formation de solutions colloïdales de nanoparticules métalliques, lesdites nanoparticules métalliques à polysaccharides réduits étant dispersées de manière homogène.

17. Procédé de préparation selon la revendication 16 comprenant en outre l'ajout d'une solution aqueuse d'un agent réducteur exogène à une concentration comprise entre 0,05 mM et 10 mM.

18. Procédé de préparation selon la revendication 16, dans lequel les concentrations de polysaccharides sont comprises entre 0,05 (p/v) et 1 % (p/v) et les concentrations de sels sont comprises entre 1 mM et 14 mM.

19. Dispositifs comprenant des matériaux nanocomposites selon l'une des revendications 1 à 8 destinés à être utilisés dans le traitement d'infections de la peau, des muqueuses et des tissus épithéliaux, intacts ou lésés, de différents sites anatomiques.
